# EUROPÄISCHE PATENTSCHRIFT

(11) **EP 1 296 701 B1**
(45) Veröffentlichungstag und Bekanntmachung des Hinweises auf die Patenterteilung: **30.09.2009**
(21) Anmeldenummer: 01960404.0
(22) Anmeldetag: 27.06.2001
(51) Int. Cl.: A61P 17/00, A61Q 17/00, A61K 36/07

(54) **VERWENDUNG VON EXTRAKTEN DES PILZES GRIFOLA FRONDOSA**
USE OF GRIFOLA FRONDOSA FUNGUS EXTRACTS
UTILISATION D'EXTRAITS DU CHAMPIGNON GRIFOLA FRONDOSA

(30) Priorität: 06.07.2000 EP 00440209
(43) Veröffentlichungstag der Anmeldung: 02.04.2003
(60) Teilanmeldung: 07009137.6
(73) Patentinhaber: Cognis France, S.A.S., 31360 Boussens (FR)
(72) Erfinder: HENRY, Florence, F-54600 Villers-les-Nancy (FR); DANOUX, Louis, F-54420 Saulxures-les-Nancy (FR); PAULY, Gilles, F-54000 Nancy (FR)
(74) Vertreter: Fabry, Bernd
(86) Internationale Anmeldenummer: PCT/EP2001/007335
(87) Internationale Veröffentlichungsnummer: WO 2002/002129

(56) Entgegenhaltungen:
- EP-A- 0 413 053
- DATABASE WPI Section Ch, Week 199952 Derwent Publications Ltd., London, GB; Class B04, AN 1999-604899 XP002149415 & JP 11 263732 A (ICHIMARU PHARCOS INC), 28. September 1999 (1999-09-28)
- DATABASE WPI Section Ch, Week 199402 Derwent Publications Ltd., London, GB; Class B04, AN 1994-010991 XP002149416 & JP 05 317016 A (TANABE SEIYAKU CO), 3. Dezember 1993 (1993-12-03)
- DATABASE WPI Section Ch, Week 199013 Derwent Publications Ltd., London, GB; Class D21, AN 1990-096548 XP002149417 & JP 02 049710 A (NARIS KESHOHIN KK), 20. Februar 1990 (1990-02-20) in der Anmeldung erwähnt
- DATABASE WPI Section Ch, Week 199025 Derwent Publications Ltd., London, GB; Class D16, AN 1990-188374 XP002149418 & JP 02 121905 A (NARIS KESHOHIN KK), 9. Mai 1990 (1990-05-09)
- PATENT ABSTRACTS OF JAPAN vol. 1999, no. 14, 22. Dezember 1999 (1999-12-22) & JP 11 263732 A (ICHIMARU PHARCOS CO LTD), 28. September 1999 (1999-09-28)
- PATENT ABSTRACTS OF JAPAN vol. 1995, no. 02, 31. März 1995 (1995-03-31) & JP 06 312937 A (YUKIGUNI MAITAKE:KK), 8. November 1994 (1994-11-08)
- DATABASE WPI Section Ch, Week 199119 Derwent Publications Ltd., London, GB; Class B04, AN 1991-136045 XP002149419 & JP 03 072428 A (HATAKEYAMA E), 27. März 1991 (1991-03-27)
- PATENT ABSTRACTS OF JAPAN vol. 018, no. 322 (C-1214), 20. Juni 1994 (1994-06-20) & JP 06 072889 A (KONPETSUKUSU:KK), 15. März 1994 (1994-03-15)
- DATABASE WPI Section Ch, Week 200017 Derwent Publications Ltd., London, GB; Class D13, AN 2000-189106 XP002149420 & JP 2000 032924 A (AKAMA A), 2. Februar 2000 (2000-02-02)

## Beschreibung

### Gebiet der Erfindung

Die Erfindung befindet sich auf dem Gebiet der kosmetischen und dermatologischen Pflegemittel und betrifft die kosmetische and therepeutische Verwendung von Extrakten des Pilzes Grifola frondosa.

### Stand der Technik

Es ist bekannt, dass Extrakte aus höheren, essbaren Pilzen, den sogenannten Basidomyceten in der Medizin wichtige Therapiemöglichkeiten bieten. So werden Extrakte von essbaren Pilzen eingesetzt zur Therapie bei AIDS-Erkrankung oder auch zur anti-Tumor-Therapie. Die Verwendung dieser Extrakte wird auch bei Diabetes und gegen Fettleibigkeit erfolgreich praktiziert. Bei den genannten essbaren Pilzen aus der Familie der Basidomyceten und der Art Agaricales (Blätterpilze) handelt es sich z.B. um Agaricus campester (Feldchampignon), Agaricus blazei, Panellus serotinus, Grifola frondosa (Maitake) oder Tremella fuciformis.

Einige kosmetische Zubereitungen enthalten eine Kombination aus Extrakten vieler einzelner höherer Pilze und finden z. B. Anwendung als skin whitener (JP 02049710) oder auch als antiallergisches Mittel (JP 1228480). In diesen Schriften wird jedoch nicht klar dargestellt, welcher der Pilzextrakte für die jeweilige Wirkung verantwortlich ist und welcher Extrakt für diese Art der kosmetischen Anwendung den essentiellen Bestandteil in der kosmetischen Zubereitung darstellt.

Kosmetische Zubereitungen stehen dem Verbraucher heute in einer Vielzahl von Kombinationen zur Verfügung. Dennoch besteht im Markt das Bedürfnis nach Produkten mit einem verbesserten Leistungsspektrum. Hierbei sind Hautverträglichkeit sowie der Einsatz natürlicher Produkte beim Kunden gefragt. Für die Herstellung von Produkten, die gleichzeitig eine Vielzahl von Anwendungen erlauben, besteht bisher das Problem, dass ihren Zubereitungen eine große Zahl an Wirkstoffen zugesetzt werden müssen, die gemeinsam das gewünschte Anforderungsprofil ergeben, ohne sich dabei gegenseitig zu stören oder gar unerwünschte Nebeneffekte zu erzeugen. Dem entsprechend besteht ein besonderes Interesse an Pflegemitteln, die die gewünschten Eigenschaften in sich vereinigen. Daneben ist es wünschenswert durch das Auffinden neuer Einsatzgebiete bereits bekannter Substanzklassen deutlich bessere Produkte zu erhalten. Besonders Extrakte von nachwachsenden Rohstoffen und deren Inhaltstoffe finden immer häufiger Einsatz in der Kosmetik.

### Beschreibung der Erfindung

Die Aufgabe der vorliegenden Patentanmeldung hat darin bestanden, Extrakte aus nachwachsenden Rohstoffen für die kosmetische und/oder dermatologische Anwendung zur Verfügung zu stellen, die in JP 11 263732 A (Ichimaru Pharcos Inc.), 28. September 1999, offenbart die Verwendung von Grifola frondosa-Extrakten als Feuchtigkeitsspender in Kosmetika. JP 02 049710 A (Naris Keshohin KK), 20. Februar 1990, offenbart die Verwendung von Grifola-frondosa-Extrakten als Feuchtigkeitsspender und Hemmstoffe der Tyrosinase. JP 02 121905 A (Naris Keshohin KK), 9. Mai 1990, offenbart die Verwendung von Grifola-frondosa-Extrakten als Hemmstoffe der Tyrosinase. großen Mengen zugänglich sind und die eine vielseitige Anwendung als Pflegemittel in den unterschiedlichsten Bereichen der Kosmetik und/oder Dermatologie ermöglichen.

Eine weitere Aufgabe der vorliegenden Patentanmeldung hat darin bestanden, Extrakte aus höheren Pilzen als nachwachsende Rohstoffe zu finden, die einen Einsatz in der Kosmetik und/oder Dermatologie begründen.

Überraschenderweise wurde gefunden, dass durch die Verwendung von Extrakten aus Grifola frondosa Pflegemittel erhalten werden, die gleichzeitig eine Vielzahl von guten pflegenden und schützenden Eigenschaften für die Haut aufweisen, sowie außerdem eine hohe Hautverträglichkeit besitzen.

Die erfindungsgemäßen mehrfachen Einsatzgebiete des Extraktes aus dem nachwachsendem Rohstoff Grifola frondosa machen ihn für den Markt und für den Verbraucher sehr attraktiv. Die komplexe Aufgabe der Erfindung konnte somit durch die Verwendung von Extrakten aus Grifola frondosa gelöst werden.

Unter dem Begriff nachwachsende Rohstoffe im Sinne der vorliegenden Anmeldung sind sowohl ganze höhere Pilze, als auch deren Bestandteile (Fruchtkörper, Stiel, Mycel) sowie deren Gemische zu verstehen. Besonders bevorzugt zur Extraktion des Pilzes im Sinne der Erfindung sind die Fruchtkörper.

### Grifola frondosa

Die erfindungsgemäß einzusetzenden Extrakte werden aus höheren Pilzen der Gattung Basidomyceten gewonnen und zwar handelt es sich speziell um Extrakte des essbaren Pilzes Grifola frondosa, der auch als Maitake bezeichnet wird. Bei diesem Pilz handelt es sich um sogenannte Ständerpilze, die auch als Basidienpilze bezeichnet werden. Diese Pilze bieten einen großen Vorteil, da sie in großen Mengen kultivierbar sind. Die Verfügbarkeit ist sehr hoch und von den Jahreszeiten unabhängig.

### Extraktion

Die Herstellung der erfindungsgemäß einzusetzenden Extrakte erfolgt durch übliche Methoden der Extraktion. Bezüglich der geeigneten herkömmlichen Extraktionsverfahren wie der Mazeration, der Remazeration, der Digestion, der Bewegungsmazeration, der Wirbelextraktion, Ultraschallextraktion, der Gegenstromextraktion, der Perkolation, der Reperkolation, der Evakolation (Extraktion unter vermindertem Druck), der Diakolation und Festflüssig-Extraktion unter kontinuierlichem Rückfluß, die in einem Soxhlet-Extraktor durchgeführt wird, die dem Fachmann geläufig und im Prinzip alle anwendbar sind, sei beispielhaft auf Hagers Handbuch der Pharmazeutischen Praxis, (5. Auflage, Bd. 2, S. 1026-1030, Springer Verlag, Berlin-Heidelberg-New-York 1991) verwiesen. Als Ausgangsmaterial können frische oder getrocknete Pilze oder Pilzbestandteile eingesetzt werden, bevorzugt ist der Einsatz von getrockneten Pilzen oder Pilzbestandteilen, üblicherweise wird jedoch von Pilzen oder Pilzbestandteilen ausgegangen, die vor der Extraktion mechanisch zerkleinert werden. Hierbei eignen sich alle dem Fachmann bekannten Zerkleinerungsmethoden, als Beispiel sei die Zerstoßung mit einem Mörser genannt.

Als Lösungsmittel für die Durchführung der Extraktionen können vorzugsweise Wasser, organische Lösungsmittel oder Gemische aus organischen Lösungsmitteln und Wasser, insbesondere niedermolekulare Alkohole, Kohlenwasserstoffe, Ketone, Ester oder halogenhaltige Kohlenwasserstoffe mit mehr oder weniger hohen Wassergehalten (destilliert oder nicht destilliert) vorzugsweise wässrig, alkoholische Lösungen einer Temperatur von größer oder gleich 20 °C verwendet werden. Besonders bevorzugt ist die Extraktion mit Wasser, Methanol, Ethanol, Hexan, Cyclohexan, Pentan, Aceton, Propylenglycolen, Polyethylenglycolen, Ethylacetat, Dichlormethan, Trichlormethan sowie Mischungen hieraus. Die Extraktion erfolgt in der Regel bei 20 bis 100 °C, bevorzugt bei 20 bis 85°C, insbesondere entweder bei Siedetemperatur des verwendeten Lösungsmittels oder bei Raumtemperatur. In einer möglichen Ausführungsform erfolgt die Extraktion unter Inertgasatmosphäre zur Vermeidung der Oxidation der Inhaltsstoffe des Extraktes. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem gewünschten Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion getrockneter Pilze oder getrockneter Pilzbestandteile gegebenenfalls entfettet, liegen im Bereich von 1,5 bis 25, insbesondere 1,9 bis 20,3 Gew.-%. Die vorliegende Erfindung umfasst die Erkenntnis, dass die Extraktionsbedingungen sowie die Ausbeuten der Endextrakte je nach gewünschtem Einsatzgebiet gewählt werden können. Falls gewünscht, können die Extrakte anschließend beispielsweise einer Sprüh- oder Gefriertrocknung unterworfen werden.

Die Einsatzmenge der Pilzextrakte in den genannten Zubereitungen richtet sich nach der Art der Anwendungen der Extrakte und nach der Konzentration der einzelnen Inhaltstoffe. Die Gesamtmenge des Pilzextraktes, der in den erfindungsgemäßen Zubereitungen enthalten ist, beträgt in der Regel 0,001 bis 25 Gew.-%, vorzugsweise 0,03 bis 5 Gew.-%, insbesondere 0,03 bis 0,1 Gew.-% bezogen auf die Endzubereitung, mit der Maßgabe, dass sich die Mengenangaben mit Wasser und gegebenenfalls weiteren Hilfs- und Zusatzstoffen zu 100 Gew.-% addieren.

Der Gesamtanteil der Hilfs- und Zusatzstoffe kann 1 bis 50, vorzugsweise 5 bis 40 Gew.-% - bezogen auf die Endzubereitung der kosmetischen und/oder pharmazeutischen Zubereitungen - betragen. Die Herstellung der Zubereitungen kann durch übliche Kalt - oder Heißprozesse erfolgen; vorzugsweise arbeitet man nach der Phaseninversionstemperatur-Methode.

Die Begriffe Zubereitungen, Endzubereitungen und Mittel sind im Sinne der Erfindung mit dem Begriff Pflegemittel gleichzusetzen.

Aktivsubstanz im Sinne der Erfindung bezieht sich auf den Anteil an Substanzen sowie Hilfs- und Zusatzstoffen, die in den Zubereitungen enthaltend sind, mit Ausnahme des zusätzlich hinzugefügten Wassers.

### Pflegemittel:

Als Pflegemittel im Sinne der Erfindung sind Pflegemittel für die Haut zu verstehen. Diese Pflegemittel schließen unter anderem stimulierende, heilende und aufbauende Wirkung für die Haut ein. Als Pflegemittel im Sinne der Erfindung sind bevorzugt solche Pflegemittel zu verstehen, die einen stimulierenden Effekt auf die Hautzellen und deren Funktionen besitzen sowie weiterhin aufbauende Wirkung für die Haut und vorbeugende Wirkung gegen Umwelteinflüsse für die Haut zeigen. Weiterhin sind im Sinne der Erfindung bevorzugt Pflegemittel als solche zu verstehen, die verschiedene Erkrankungen der Haut mit ihren unterschiedlichen Auswirkungen auf Aussehen und Funktion der Haut entweder verbessern oder heilen können.

Die erfindungsgemäßen Zubereitungen zeigen eine hervorragende hautpflegende Wirkung bei gleichzeitig hoher Hautverträglichkeit. Außerdem zeigen sie eine gute Stabilität, insbesondere gegenüber oxidativer Zersetzung der Produkte.

### Dermale Makromoleküle

Als dermale Makromoleküle sind im Sinne der Erfindung prinzipiell alle Makromoleküle zu verstehen, die als Bestandteile der Haut entweder in der Basalmembran zwischen Dermis und Epidermis oder in der Dermis und Epidermis direkt zu finden sind. Es handelt sich im Besonderen um Verbindungen die ausgewählt sind aus der Gruppe, die gebildet wird von Collagen, Elastin, Proteoglycanen, Fibronectinen und Hyaluronsäure und deren Salze. Collagen besteht aus Proteinfasern und kommt in menschlicher Haut in drei verschiedenen Typen (Typ I, III und IV) vor. Im Collagen sind die einzelnen Polypeptidketten, die jeweils viel von der Aminosäure Prolin und als jeden dritten Rest Glycin, umeinander zu einer Tripelhelix gewunden. Die Collagenfasern werden als Tropokollagen in den Fibroblasten synthetisiert und in die extrazelluläre Matrix ausgeschleust. Die erfindungsgemäße Stimulierung der Synthese des Collagens führt zu einer Erhöhung der Produktion an Collagen und damit zu einer erhöhten intermolekularen Verfestigung der Dermis und dadurch zu einer straffer erscheinenden Haut. Das Elastin ist ebenfalls ein faserartiges Protein. Hierbei handelt es sich um unstrukturierte kovalent quervernetzte Polypeptidketten, die ein gummiähnliches elastisches Material bilden. Das Elastin wird nach der Synthese in den Hautzellen in die extrazelluläre Matrix ausgeschleust. Die Stimulierung der Synthese der Elastin-Polypeptidketten führt zu einer Erhöhung der Produktion an Elastin und damit zu einer Erhöhung der Elastizität der Haut.

Die **Proteoglycane** bestehen wie die Glycoproteine aus Kohlenhydraten und aus Proteinen, bei den Proteoglycanen überwiegt jedoch der Anteil an Polysacchariden. Die Proteoglycane der Haut enthalten Dermatansulfat. Es lagern sich ca. 140 solcher Proteoglycane mit Hilfe kleinerer Proteine (Link-Proteine) nichtkovalent an eine Hyaluronsäure-Kette zu Molekül-Aggregaten mit einer mittleren Molmasse von ca. 2 Mio. an. Die durch ihr Wasserbindevermögen ausgezeichneten polyanionischen Aggregate können feste Gele bilden, die dem Stützgewebe (extrazelluläre Matrix) Elastizität und Zugfestigkeit verleihen. In Schleimen schützen sie die Epithelien. Die Stimulierung der Synthese von Proteoglycanen und Hyaluronsäure führt zu einer größeren Menge an extrazellulärer Matrix und damit zu einer erhöhten Elastizität und Zugfestigkeit.

**Fibronectin** stellt eine Gruppe hochmolekularer Glykoprotein (MR des Dimers ca. 440 000-550 000) dar, die sich in der extrazellulären Matrix und in extrazellulären Flüssigkeiten finden. Das durch zwei Disulfid-Brücken verbundene Fibronectin-Dimer, ein langgestrecktes Molekül mit den Abmessungen 600×25 Å, bindet durch lineare Kombination dreier verschiedener sich wiederholender Domänen u. a. Collagene, Glykosaminoglykane, Proteoglykane, Fibrin(ogen), Desoxyribonucleinsäuren, Immunglobuline, Plasminogen, Plasminogen-Aktivator, Thrombospondin, Zellen und Mikroorganismen. Durch diese Eigenschaften vermittelt es z. B. die Anhaftung von Bindegewebszellen an Collagen-Fibrillen oder von Thrombocyten und Fibroblasten an Fibrin (Beitrag zur Wundheilung).

Die **Hyaluronsäure** ist ein saures Glykosaminoglykan, Grundbaustein der Hyaluronsäure ist ein aus D-Glucuronsäure und N-Acetyl-D-glucosamin in (beta 1-3)-glykosidischer Bindung aufgebautes Aminodisaccharid, das mit der nächsten Einheit (beta 1-4)-glykosidisch verbunden ist.

Die erfindungsgemäßen Extrakte können weiterhin in kosmetischen und/oder dermatologischen Pflegemitteln zur Verminderung der Proteolyse und Glycation von Makromolekülen in der Haut verwendet werden. Bei der **Proteolyse** handelt es sich um einen Prozess, bei dem es zu einer Spaltung von Proteinen durch Hydrolyse der Peptid-Bindungen mittels Säuren oder Enzymen kommt.

Eine andere Bezeichnung ist die Proteinase Verdauung. Die erfindungsgemäße Verminderung der Proteolyse führt zu einer verminderten Spaltung der dermalen Makromoleküle, die eine Proteinstruktur aufweisen und damit zur Verhinderung der Verminderung einer Hautfestigung und zur Verhinderung des Abbaus einer erhöhten Elastizität. Die **Glycation** ist eine nichtenzymatische Reaktion von Glucose oder anderen Zuckern mit Proteinen zu Glycoproteinen. Diese Reaktion führt zu unbeabsichtigten Veränderungen am Collagen und Elastin und damit zu Veränderungen der extrazellulären Matrix. Die Funktion des Collagens und der extrazellulären Matrix sind gestört. Die Verhinderung der Glycation führt zu einer Verringerung der nichtenzymatischen Veränderung an Collagen und Elastin und damit zur Verhinderung einer verminderten Funktion der extrazellulären Matrix.

Ein weiterer Gegenstand der Erfindung ist die Verwendung von Extrakten aus Grifola frondosa in Pflegemitteln zur vorbeugenden oder heilenden Behandlung von Alterserscheinungen der Haut. Eine andere Bezeichnung für diese Art der Pflegemittel ist auch anti-ageirig Mittel. Zu diesen Alterserscheinungen zählen beispielsweise jede Art der Fältchen- und Faltenbildung. Die Behandlungen schließen eine Verlangsamung von Altersprozessen der Haut mit ein. Die Alterserscheinungen können die unterschiedlichsten Ursachen aufweisen. Insbesondere sind diese Alterserscheinungen auf Grund einer durch UV-Strahlung induzierten Schädigung der Haut verursacht. In einer besonderen Ausführungsform der Erfindung werden diese Pflegemittel zur Behandlung von durch UV-Strahlung induzierten Alterserscheinungen der Haut eingesetzt.

Ein weiterer Gegenstand der Erfindung ist Verwendung von Extrakten des Pilzes Grifola frondosa in kosmetischen und/oder dermatologischen Sonnenschutzmitteln.

Als Sonnenschutzmittel bzw. UV-Lichtschutzfaktoren im Sinne der Erfindung werden Lichtschutzmittel bezeichnet, die für den Schutz der menschlichen Haut gegenüber schädigenden Einflüssen der direkten und indirekten Strahlung der Sonne nützlich sind. Die für die Hautbräunung verantwortliche Ultraviolettstrahlung der Sonne unterteilt man in die Abschnitte UV-C (Wellenlängen 200-280 nm), UV-B (280-315 nm) und UV-A (315-400 nm).

Die Pigmentierung normaler Haut unter dem Einfluss der Sonnenstrahlung, d. h. die Bildung von Melaninen, wird durch UV-B u. UV-A unterschiedlich bewirkt. Bestrahlung mit UV-A-Strahlen ("langwelligem UV") hat die Dunkelung der in der Epidermis bereits vorhandenen Melanin-Körper zur Folge, ohne dass schädigende Einflüsse zu erkennen sind. Anders bei dem sog. "kurzwelligen UV" (UV-B). Dieses bewirkt die Entstehung von sog. Spätpigment durch Neubildung von Melanin-Körnern. Ehe jedoch das (schützende) Pigment gebildet ist, unterliegt die Haut der Einwirkung der ungefilterten Strahlung, die - je nach Expositionsdauer - zur Bildung von Hautrötungen (Erythemen), Hautentzündungen (Sonnenbrand) und gar Brandblasen führen kann.

Als UV-Absorber oder Lichtfilter, die also die UV-Strahlung in unschädliche Wärme umwandeln, werden die erfindungsgemäßen Extrakte des Pilzes Grifola frondosa eingesetzt, diese können zusätzlich in Kombination mit weiteren Sonnenschutzmitteln bzw. UV-Lichtschutzfaktoren vorliegen.

Diese weiteren UV- Lichtschutzfaktoren sind beispielsweise bei Raumtemperatur flüssig oder kristallin vorliegende organische Substanzen (Lichtschutzfilter), die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der EP 0693471 B1 beschrieben;
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-isopropylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UVA-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beispielsweise beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Dimethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122,543 (1996**)** sowie Parfümerie und Kosmetik 3 (1999), Seite 11ff zu entnehmen.

Die erfindungsgemäßen Extrakte können weiterhin in kosmetischen und/oder dermatologischen Pflegemitteln als Tyrosinaseinhibitoren und/oder als Hautweißungsmittel verwendet werden. Die auch als Skin-whitener bezeichneten Hautweißungsmittel führen zu einem helleren Aussehen der Haut. Eine Möglichkeit zur Hautaufhellung oder Hautweissung führt über die Inhibierung der Tyrosinase, denn die Tyrosinase ist an der Bildung des Hautpigments Melanin beteiligt (Depigmentierung). Der Einsatz von Extrakten aus Grifola frondosa führt auf Grund der Inhibierung der Tyrosinase zu einer verminderten Bildung von Melanin und damit zu einer Hautweißung. Die Extrakte aus Grifola frondosa können zusätzlich in Kombination mit weiteren Tyrosinaseinhibitoren als Depigmetierungsmittell wie beispielsweise Arbutin, Ferulasäure, Kojisäure, Cumarinsäure und Ascorbinsäure (Vitamin C) eingesetzt werden.

Die Verwendung der Extrakte als schützende und aufbauende Pflegemittel ist prinzipiell für alle Zubereitungen möglich, die zur Prävention gegen Schädigungen oder bei Schädigungen der Haut und damit in der Hautpflege eingesetzt werden. Eine andere Verwendung auf diesem Gebiet ist die Applikation bei empfindlicher, durch Allergie oder anderen Ursachen geschädigter Haut. Die Schädigung der Haut kann dabei unterschiedlichste Ursachen haben.

Prinzipiell kann man die erfindungsgemäßen Extrakte in allen kosmetischen Produkten einsetzen. Beispiele für kosmetische Produkte sind in ihren Formulierungen in den Tabelle 5 bis 8 beschrieben.

### Kosmetische und/oder dermatologische Zubereitungen

Die erfindungsgemäßen Zubereitungen können zur Herstellung von kosmetischen und/oder dermatologischen Zubereitungen, wie beispielsweise Schaumbäder, Duschbäder, Cremes, Gele, Lotionen, alkoholische und wäßrig/alkoholische Lösungen, Emulsionen, Wachs/ Fett-Massen, Stiftpräparaten, Pudern oder Salben dienen. Diese Mittel können ferner als weitere Hilfs- und Zusatzstoffe milde Tenside, Ölkörper, Emulgatoren, Perlglanzwachse, Konsistenzgeber, Verdikkungsmittel, Überfettungsmittel, Stabilisatoren, Polymere, Siliconverbindungen, Fette, Wachse, Lecithine, Phospholipide, biogene Wirkstoffe, Antioxidantien, Deodorantien, Antitranspirantien, Filmbildner, Quellmittel, Insektenrepellentien, Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. amphotere Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester,

Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quatemierte Fettsäuretrialkanolaminestersalze. Typische Beispiele für amphotere bzw. zwitterionische Tenside sind Alkylbetaine, Alkylamidobetaine, Aminopropionate, Aminoglycinate, lmidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise J.Falbe (ed.), "Surfactants in Consumer Products", Springer Verlag, Berlin, 1987, S. 54-124 **oder** J.Falbe (ed.), "Katalysatoren, Tenside und Mineralöladditive", Thieme Verlag, Stuttgart, 1978, S. 123-217 verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, lsostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. DE 19756377 A1), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Trglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestem mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol gemäß DE 1165574 PS und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE 2024051 PS als Rückfettungsmittel für kosmetische Zubereitungen bekannt.

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 lsostearate (Isolan® Gl 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispiels-weise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquatemierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B.

Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quatemierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der FR 2252840 A sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobomylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tertButylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in Cosm.Toil.108, 95 (1993**)** aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm.Toil. 91, 27 (1976**).**

### Antioxidantien

Neben primären Lichtschutzstoffen können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilat, Irotyl und Floramat allein oder in Mischungen, eingesetzt.

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
➢ adstringierende Wirkstoffe,
➢ Ölkomponenten,
➢ nichtionische Emulgatoren,
➢ Coemulgatoren,
➢ Konsistenzgeber,
➢ Hilfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zrko-nium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993**)** entnommen werden.

### Insekten-Repellentien

Als Insekten-Repellentien kommen N,N-Diethyl-m-toluamid, 1,2-Pentandiol oder Ethyl Butylacetylaminopropionate in Frage

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

### Parfümöle

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

### Farbstoffe

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

### Beispiele

### 1. Beispiel: Extraktion der Pilze mit wässrigem Ethanol

300 g getrocknete Grifola frondosa Pilze wurden in einen Glasreaktor mit 3 l 96 Gew.-%-igem wässrigen Ethanol überführt. Der Aufguß wurde unter Rühren über einen Zeitraum von 1 h bei Siedetemperatur extrahiert. Anschließend wurde die Mischung auf 20 °C abgekühlt und die überstehende kolloide Flüssigkeit wurde durch Filtration an Tiefenfilter mit einer mittleren Porosität von 450 nm (von der Firma Seitz, Bordeaux Frankreich) vom Rückstand getrennt. Anschließend wurde zunächst der Alkohol bei 45 °C unter vermindertem Druck entfernt und dann der Rückstand bei 50 °C getrocknet. Die Ausbeute an Trockenprodukt betrug 10,9 Gew.-% bezogen auf das Trockengewicht an eingesetzten Pilzen.

### 2. Beispiel: Extraktion der Pilze mit wässrigem Ethanol und Wasser

Beispiel 1 wurde wiederholt, 100 g des Trockenproduktes jedoch anschließend in einen Glasreaktor mit 1 l einer wässrigen Lösung mit einem pH Wert von 7.5 überführt. Der pH Wert wurde mit einer 4 N NaOH-Lösung eingestellt. Die Extraktion wurde unter Rühren 1 h bei Raumtemperatur durchgeführt und der Extrakt 15 min bei einer Geschwindigkeit von 5000 g zentrifugiert. Die überstehende Flüssigkeit wurde durch Filtration vom Rückstand getrennt. Die Filtration wurde wie im Beispiel 1 beschrieben durchgeführt und der Rückstand sprühgetrocknet. Die Ausbeute an Trockenprodukt betrug 20,3 Gew.-% bezogen auf das Trockengewicht an eingesetzten Pilzen. Der Anteil an Proteinen im Trockenprodukt betrug 9,2 Gew.-%. Der Anteil an Zuckern im Trockenprodukt betrug 53,8 Gew.%.

### 3. Beispiel: Extraktion der Pilze mit Hexan

Beispiel 1 wurde wiederholt, die Extraktion von 500 g getrockneten Grifola frondosa jedoch mit 5 l Hexan durchgeführt. Die Extraktion wurde unter Rühren 1 h bei Siedetemperatur unter reflux durchgeführt und der Extrakt wie beschrieben weiter verarbeitet. Die Filtration wurde wie im Beispiel 1 beschrieben durchgeführt. Anschließend wurde zunächst des Lösungsmittel unter vermindertem Druck entfernt und dann der Rückstand bei 50 °C getrocknet. Die Ausbeute an Trockenprodukt betrug 1,9 Gew.-% bezogen auf das Trockengewicht an eingesetzten Pilzen.

### 4. Analytische Untersuchung

Die Extrakte nach Beispiel 1 bis 3 wurden an einer Kationenaustauscher HPLC an einer Chromopack CP28350 (300 x 6,5) Säule bei einer Temperatur von 70 °C, einer Flußrate von 0,8ml/min, einer mobilen Phase aus einer 0,01 N H₂SO₄-Lösung und einer isokratischen Elution analysiert. Bei dem Detektor handelte es sich um einen UV Detektor mit 210 nm Wellenlänge. Es sollte der Gehalt an Fumarsäure in den einzelnen Extrakten untersucht werden Die Retentionszeit eines Standards von Fumarsäure betrug 9,29 min. Es wurden je 10 µl eines 5 Gew.-% Extraktes injiziert.

**Tabelle 1: Analytische Bestimmung des Gehaltes an Fumarsäure in den Extrakten nach Beispiel 1 bis 3**

| **Extrakt** | **Gehalt an Fumarsäure (g/100g Extrakt)** |
|---|---|
| Extrakt nach Beispiel 3 / 5-Gew.-% | 0 |
| Extrakt nach Beispiel 1 / 5-Gew.-% | 0,0025 |
| Extrakt nach Beispiel 2 / 5-Gew.-% | 0,038 |

Die Ergebnisse in der Tabelle zeigen, dass sich im Extrakt nach Beispiel 3 keine Fumarsäure nachweisen lässt und die Extrakte nach Beispiel 1 und 2 nur sehr geringe Mengen Fumarsäure enthalten.

### 5. Regenerierende und revitalisierende Aktivität auf der Haut

Das Ziel dieser Tests ist der Nachweis einer regenerierenden und revitalisierenden Aktivität von Extrakten aus Grifola frondosa an humanen Fibroblastenkulturen in vitro.

Methode 1: Effekte am Zellwachstum Humane Fibroblasten wurden in einem definiertem Nährmedium (DMEM = Dulbecco Minimum Essential Medium, Firma Life Technologie Sarl) mit 10 Gew.-% fötalem Kälberserum angeimpft und für 24 h bei 37 °C in einer 5 %igen CO₂-Atmosphäre inkubiert. Anschließend wurde das Nährmedium mit fötalem Kälberserum durch ein Nährmedium aus DMEM ohne fötalem Kälberserum ausgetauscht. Zu diesem Nährmedium wurden unterschiedliche Konzentrationen an Aktivsubstanz in Form der Extrakte aus Grifoa frondosa nach Beispiel 1 und 2 gegeben. Zum Vergleich wurde als Kontrolle eine Testreihe von humanen Fibroblasten ohne Aktivsubstanz inkubiert. Nach einer dreitägigen Inkubation der Fibroblasten im Nährmedium wurde das Wachstum und die Stoffwechselaktivität beurteilt, indem die Zellen mittels eines Partikelzählers ausgezählt wurden und indem der intrazelluläre Anteil an ATP nach der Methode von Vasseur (Journal francais Hydrologie, 1981, 9, 149-156.), der Anteil an Zellproteinen nach der Methode von Bradford (Anal. Biochem., 1976, 72, , 248-254) und der Anteil an zellularer DNA nach der Methode von Desaulniers (In vitro, 1998, 12(4), 409-422) bestimmt wurde. Bei Konzentrationen von 0,001 bis 0,1 Gew.-% Pilzextrakt nach Beispiel 1 und 2 erhielt man eine Erhöhung des Anteils an ATP zwischen 8 und 23 % im Vergleich zur Kontrolle. Der Anteil an Zellproteinen erhöhte sich bei Konzentrationen von 0,001 bis 0,1 Gew.-% Pilzextrakt nach Beispiel 1 und 2 zwischen 5 und 32 % im Vergleich zur Kontrolle. Der Anteil an zellulärem DNA wurde nach 3 und nach 6 Tagen Inkubation bei Konzentrationen von 0,001 und 0,003 Gew.-% Pilzextrakt nach Beispiel 1 bestimmt und es zeigt sich, dass der Anteil zwischen 10 und 42 % im Vergleich zur Kontrolle gestiegen ist.

**Tabelle 2: Erhöhung des Proteingehaltes und des ATP-Gehaltes in humanen Fibroblasten nach Inkubation mit Extrakten nach Beispiel 1 bis 3 im Vergleich zur Inkubation ohne Pilzextrakt**

| Substanz | Konzentration Gew-% | Gehalt an Proteinen in % | Gehalt an ATP |
|---|---|---|---|
| Kontrolle | | 100 | 100 |
| Extrakt nach Beispiel 1 | 0,001 | 114 | 108 |
| | 0,003 | 132 | 123 |
| | 0,01 | 126 | 81 |
| Extrakt nach Beispiel 2 | 0,001 | 115 | 113 |
| | 0,003 | 105 | 118 |
| | 0,01 | 98 | 116 |
| | 0,03 | 88 | 121 |
| Extrakt nach Beispiel 3 | 0,0001 | 108 | 104 |
| | 0,0003 | 119 | 100 |
| | 0,001 | 136 | 108 |
| | 0,003 | 129 | 86 |

**Tabelle 3: Erhöhung des DNA-Gehaltes in humanen Fibroblasten nach Inkubation mit Extrakten nach Beispiel 1 im Vergleich zur Inkubation ohne Pilzextrakt**

| Substanz | Konzentration Gew-% | 3 Tage Inkubation in % | 6 Tage Inkubation |
|---|---|---|---|
| Kontrolle | | 100 | 100 |
| Extrakt nach Beispiel 1 | 0,001 | 128 | 138 |
| | 0,003 | 110 | 142 |

Die Studie zeigt, dass die Extrakte aus Grifola frondosa nach Beispiel 1 bis 3 das Wachstum und den Stoffwechsel (Metabolismus) der humanen Fibroblasten in vitro erheblich anregt.

Methode 2: Verbesserung der Überlebensfähigkeit Die Test wurden durchgeführt an humanen Fibroblasten. Der Test ermöglicht auf den ruhenden Zellen eine gewisse Anzahl von Parametern mengenmäßig zu bestimmen. Die Kultivierung der Zellen entspricht der Kultivierung aus der Methode 1, ausgenommen der Inkubationszeit. Die Inkubationszeit für diese Test betrugen 72 h. Die Überlebensfähigkeit wurde beurteilt über die kolorimetische Bestimmung des Gehaltes an Proteinen nach der Methode von Bradford (Anal. Biochem. 1976, 72, 248-254.) Die Test wurden dreifach durchgeführt und dann zweimal wiederholt, so dass es sechs Ergebnisse pro Pilzextrakt gab, die jeweils gemittelt wurden. Die Ergebnisse wurden ermittelt in Prozent im Vergleich zur Kontrolle.

**Tabelle 4: Erhöhung des Proteingehaltes in humanen Fibroblasten nach Inkubation mit Extrakten nach Beispiel 1 bis 3 im Vergleich zur Inkubation ohne Pilzextrakt**

| Substanz | Konzentration Gew-% | Gehalt an Proteinen in % |
|---|---|---|
| Kontrolle | | 100 |
| Extrakt nach Beispiel 1 | 0,001 | 121 |
| | 0,003 | 135 |
| | 0,01 | 180 |
| Extrakt nach Beispiel 2 | 0,001 | 115 |
| | 0,003 | 111 |
| | 0,01 | 126 |
| Extrakt nach Beispiel 3 | 0,001 | 105 |
| | 0,003 | 112 |
| | 0,01 | 125 |

Die Extrakte von Grifola frondosa nach Beispiel 1 bis 3 in Konzentrationen von 0,001 bis 0,01 Gew.-% erhöhen den Gehalt an Proteinen in humanen Fibroblasten im Vergleich zur Kontrolle um 5 bis 80 % bei in vitro Untersuchungen. Diese Ergebnisse zeigen, dass die Extrakte von Grifola frondosa eine hohe Kapazität besitzen um den Metabolismus von Fibroblasten anzuregen. Die Extrakte zeigen eine regenerierende und revitalisierende Aktivität an humanen Fibroblasten und sind damit als Energielieferanten und als anti-ageing Mittel in kosmetischen und in dermatologischen Zubereitungen einsetzbar.

### 6. Anti-Inflammatorische Aktivität

Hintergrund: Eine kutane Inflammation (Entzündung) kann hervorgerufen werden durch UVB Strahlung aufgrund der Stimulierung epidermaler Keratinocyten. Anschliessend setzt eine akute Leukozyten Infiltration ein.

Diese Aktivierung dieser Leukozyten, speziell neutrophile Granulocyten, ist bekannt als "respiratory burst" und kann eine Gewebezerstörung vermitteln durch freigesetzten reaktiven Sauerstoffradikalen (reactive oxygen species - ROS) und durch lysosomale Enzyme.

Methode: Die anti-inflammatorische Aktivität wurde an einer Zelllinien humaner Leukocyten (neutrophile Granulocyten) untersucht. Zu diesem Zweck wurden die Zellen mit unterschiedlichen Konzentrationen an zu testenden Extrakten gemäß Beispiel 1 und 3 inkubiert und anschließend ein "respiratory burst" durch Hefezellenextrakt ("Zymosan") induziert. Der Gehalt an intermediär gebildeten und an ausgeschütteten Sauerstoffradikalen wurde durch Reaktion mit Luminol über die Lumineszenz nachgewiesen und quantitativ bestimmt. In Gegenwart von Substanzen mit radikalfangenden Eigenschaften vermindert sich die Lumineszenzausbeute. Diese Ergebnisse sind in Tabelle 4 zusammengefasst; der Anteil an freigesetzten Radikalen ist in %-absolut angegeben ("Luminol-Test"). Zur Kontrolle wurde jeweils die Anzahl der intakten Zellen mit einem Partikelzähler bestimmt und in % im Vergleich zur Kontrolle angegeben.

**Tabelle 5: Bestimmung der freigesetzten Sauerstoffradikale**

| Substanz | Konzentration Gew-% | Gehalt an ROS (%/Kontrolle) in % | Anzahl intakter Zellen |
|---|---|---|---|
| Kontrolle | | 100 | 100 |
| Extrakt nach Beispiel 1 | 0,001 | 160 | 101 |
| | 0,01 | 187 | 104 |
| | 0,1 | **68** | 101 |
| Extrakt nach Beispiel 3 | 0,001 | **51** | 100 |

Aus den obigen Ergebnissen lässt sich entnehmen, dass die erfindungsgemäßen Extrakte nach Beispiel 1 bei einer Konzentration von 0,1 % einen starken anti-inflammatorischen Effekt zeigen. Die erfindungsgemäßen Extrakte nach Beispiel 3 zeigen bereits bei einer Konzentration von 0,001 % einen starken anti-inflammatorischen Effekt. Diese Effekte auf humane Leukozyten konnten erzielt werden, ohne eine toxikologische Wirkung auf diese Zellen zu zeigen. In Verbindung mit den Ergebnissen aus Beispiel 4 lässt sich entnehmen, dass die Extrakte mit den starken anti-inflammatorischen Effekten keine oder nur geringe Mengen Fumarsäure besitzen. Der anti-inflammatorische Effekt kann also nicht auf die Gegenwart von Fumarsäure zurückgeführt werden.

### 7. Zellschutzwirkung gegen UVB an in vitro gezüchteten menschlichen Keratinozyten

Hintergrund: UVB-Strahlen lösen durch Aktivierung eines Enzyms, nämlich Phospholipase A2 oder PLA2, welche Arachidonsäure aus den Phospholipiden der Plasmamembran entfernt, eine Entzündung (Erythem, Ödem) aus. Arachidonsäure ist die Vorstufe der Prostaglandine, die eine Entzündung und eine Zellmembranschädigung verursachen; die Prostaglandine E2 (= PGE2) werden durch die Cyclooxygenase gebildet.

Methode: Der Effekt von UVB-Strahlung wurde an Keratinocyten in vitro untersucht indem die Freisetzung des Cytoplasaenzyms LDH (Lactat Dehydrogenase) bestimmt wurde. Dieses Enzym dient als Marker für eine Zellschädigung.

Zur Durchführung der Tests wurde ein definiertes Medium, das fötales Kälberserum enthält, mit den Keratinozyten beimpft und der Pflanzenextrakt (mit Saline-Lösung verdünnt) 72 Stunden nach dem Beimpfen zugegeben.

Die Keratinozyten wurden sodann mit einer UVB-Dosis bestrahlt (50 mJ/cm²- Röhren: DUKE FL40E). Nach weiterer 1 tägiger Inkubation bei 37 °C und bei 5 % CO₂ wurde der LDH-Gehalt im Überstand bestimmt. Der Gehalt von LDH- (Lactatdehydrogenase) wurde mittels einer Enzymreaktion bestimmt (verwendetes kit zur Untersuchung des LDH Gehaltes von der Firma Roche). Die Anzahl adhärenter Keratinozyten wird (nach Trypsinbehandlung) mit einem Partikelzählgerät bestimmt.

**Tabelle 6: Zellschutzwirkung von Extrakten aus Grifola frondosa gegen UVB-Strahlen; Ergebnisse in % bezogen auf die Kontrolle, Mittelwert aus 2 Versuchen, jeder mit zwei Wiederholungen**

| | Anzahl Keratinocyten | Gehalt an freigesetzte LDH |
|---|---|---|
| Kontrolle ohne UV | 100 | 0 |
| Kontrolle mit UVB (50 mJ/cm²) | 19 | 100 |
| UVB+Extrakt nach Beispiel 1/0,003 Gew.-% | 23 | 74 |
| UVB + Extrakt nach Beispiel 2/0,03 Gew.-% | 35 | 49 |
| UVB + Aspirin® / 0,03 Gew.-% (Fa. SIGMA) | | |

Die Ergebnisse dieser Tests belegen, dass ein erfindungsgemäßer Extrakt von Grifola frondosa den Effekt von UVB-Strahlung auf die Anzahl an Keratinocyten und auf den Gehalt an freigesetzte LDH reduziert. Die beschriebenen Extrakte zeigen demnach die Fähigkeit, die durch UVB-Strahlung hervorgerufene Schädigung an Zellmembranen zu reduzieren.

Die Wirkungen und positiven Aktivitäten der Extrakte aus Grifola frondosa enthalten folglich eine sehr starke
- anregende (stimulierende), revitalisierende und regenerierende Aktivität auf den Stoffwechsel,
- starke anti-inflammatorische Aktivität
- Einfangen und Neutralisieren von Radikalen und reaktionsfähigen Formen des Sauerstoffs;
- Aktivität die durch UVB-Strahlung hervorgerufene Schädigung an Zellmembranen zu reduzieren.

### 8. Beispielrezepturen kosmetischer Mittel mit Grifola frondosa Extrakten

Die gemäß Beispiel 1 bis 3 erhaltenen Grifola frondosa Extrakte wurden in den folgenden erfindungsgemäßen Rezepturen K1 bis K21 sowie 1 bis 13 eingesetzt. Die so hergestellten kosmetischen Mittel zeigten gegenüber den Vergleichsrezepturen V1, V2 und V3 sehr gute hautpflegende Eigenschaften bei gleichzeitig guter Hautverträglichkeit. Darüber hinaus sind die erfindungsgemäßen Mittel stabil gegen oxidative Zersetzung.

**Tabelle 7: Softcreme Rezepturen K1 bis K7**

| (Alle Angaben in Gew.-% bez. auf das kosmetische Mitteln) | | | | | | | | |
|---|---|---|---|---|---|---|---|---|
| INCI Bezeichnung | K1 | K2 | K3 | K4 | K5 | K6 | K7 | V1 |
| Glyceryl Stearate (and) Ceteareth-12/20 (and) Cetearyl Alcohol (and) Cetyl Palmitate | 8,0 | 8,0 | 8,0 | 8,0 | 8,0 | 8,0 | 8,0 | 8,0 |
| Cetearyl Alcohol | 2,0 | 2,0 | 2,0 | 2,0 | 2,0 | 2,0 | 2,0 | 2,0 |
| Dicaprylyl Ether | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetearyllsononanoate | 3,0 | 3,0 | 3,0 | 3,0 | 3,0 | 3,0 | 3,0 | 3,0 |
| Glycerin (86 Gew.-%ig) | 3,0 | 3,0 | 3,0 | 3,0 | 3,0 | 3,0 | 3,0 | 3,0 |
| Extrakt nach Beispiel 1 bis 3 | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure 1) | | | | | | 0,5 | | |
| Panthenol | | | | | | | 0,5 | |
| Wasser | Ad 100 | | | | | | | |

**Tabelle 8: Nachtcremerezepturen K8 bis K14**

| (Alle Angaben in Gew.-% bez. auf das kosmetische Mitteln) | | | | | | | | |
|---|---|---|---|---|---|---|---|---|
| INCI Bezeichnung | K8 | K9 | K10 | K11 | K12 | K13 | K14 | V2 |
| Polyglyceryl-2 Dipolyhydroxystearate | 4,0 | 4,0 | 4,0 | 4,0 | 4,0 | 4,0 | 4,0 | 5,0 |
| Polyglyceryl-3 Diisostearate | 2,0 | 2,0 | 2,0 | 2,0 | 2,0 | 2,0 | 2,0 | 2,0 |
| Cera Alba | 2,0 | 2,0 | 2,0 | 2,0 | 2,0 | 2,0 | 2,0 | 2,0 |
| Zinc Stearate | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetaeryllsononanoate | 8,0 | 8,0 | 8,0 | 8,0 | 8,0 | 8,0 | 8,0 | 8,0 |
| Dicaprylyl Ether | 5,0 | 5,0 | 5,0 | 5,0 | 5,0 | 5,0 | 5,0 | 5,0 |
| Magnesiumsulfate | 1,0 | 1,0 | 1,0 | 1,0 | 1,0 | 1,0 | 1,0 | 1,0 |
| Glycerin (86 Gew.-%ig) | 5,0 | 5,0 | 5,0 | 5,0 | 5,0 | 5,0 | 5,0 | 5,0 |
| Extrakt nach Beispiel 1 bis 3 | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | Ad 100 | | | | | | | |

**Tabelle 9: W/O Bodylotion Rezepturen K15 bis K21**

| (Alle Angaben in Gew.-% bez. auf das kosmetische Mitteln) | | | | | | | | |
|---|---|---|---|---|---|---|---|---|
| INCI-Bezeichnung | K15 | K16 | K17 | K18 | K19 | K20 | K21 | V3 |
| PEG-7 Hydrogenated Castor Oil | 7,0 | 7,0 | 7,0 | 7,0 | 7,0 | 7,0 | 7,0 | 7,0 |
| DecylOleate | 7,0 | 7,0 | 7,0 | 7,0 | 7,0 | 7,0 | 7,0 | 7,0 |
| Cetearyllsononanoate | 7,0 | 7,0 | 7,0 | 7,0 | 7,0 | 7,0 | 7,0 | 7,0 |
| Glycerin (86 Gew.-%ig) | 5,0 | 5,0 | 5,0 | 5,0 | 5,0 | 5,0 | 5,0 | 5,0 |
| MgSO₄·7 H₂O | 1,0 | 1,0 | 1,0 | 1,0 | 1,0 | 1,0 | 1,0 | 1,0 |
| Extrakt nach Beispiel 1 bis 3 | 1,5 | 1,5 | 1,5 | 1,5 | 1,5 | 1,5 | 1,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | Ad 100 | | | | | | | |

| | | | | | | | | |
|---|---|---|---|---|---|---|---|---|
| ¹⁾ Desoxyribonucleinsäure: Molekuargewicht ca. 70000, Reinheit (bestimmt durch spektro-photometrische Messung der Absorption bei 260 nm sowie 280 nm): mindestens 1,7. | | | | | | | | |

**Tabelle 10: Rezepturen**

| Kosmetische Zubereitungen Conditioner (Wasser, Konservierungsmittel ad 100 Gew.-%) | | | | |
|---|---|---|---|---|
| **Zusammensetzung (INCI)** | **1 Gew.%** | **2 Gew. %** | **3 Gew. %** | **4 Gew. %** |
| **Texapon® NSO** Sodium Laureth Sulfate | 38,0 | 38,0 | 25,0 | - |
| **Texapon® SB 3** Disodium Laureth Sulfosuccinate | - | - | 10,0 | - |
| **Plantacare® 818** Coco Glucosides | 7,0 | 7,0 | 6,0 | - |
| **Plantacare® PS 10** Sodium Laureth Sulfate (and) Coco Glucosides | - | - | - | 20,0 |
| **Dehyton® PK 45** Cocamidopropyl Betaine | - | - | 10,0 | |
| **Lamesoft® PO 65** Coco-Glucosid (and) Glyceryl Oleate | 3,0 | | | 4,0 |
| **Lamesoft®** LMG Glyceryl Laurate (and) Potassium Cocoyl Hydrolyzed Collagen | - | 5,0 | - | |
| **Euperlan® PK 3000 AM** Glycol Distearate (and) Laureth-4 (and) Cocamidopropyl Betaine | - | 3,0 | 5,0 | 5,0 |
| Extrakt nach Beispiel 1 bis 3 | 1,0 | 1,0 | 1,0 | 1,0 |
| **Arlypon® F** Laureth-2 | 3,0 | 3,0 | 1,0 | - |
| **Sodium Chloride** | - | 1,5 | - | 1,5 |

| | | | | |
|---|---|---|---|---|
| (1-2) Duschbad, (3) Duschgel, (4) Waschlotion | | | | |

**Tabelle 10**

| **Kosmetische Zubereitungen Duschbad "Two in One" (Wasser, Konservierungsmittel ad 100 Gew.-%) - Fortsetzung** | | | | |
|---|---|---|---|---|
| **Zusammensetzung (INCI)** | **5** | **6** | **7** | **8** |
| **Texapon® NSO** Sodium Laureth Sulfate | 30,0 | 25,0 | | 25,0 |
| **Plantacare® 818** Coco Glucosides | | | | 8,0 |
| **Plantacare® 2000** Decyl Glucoside | | 8,0 | | |
| **Plantacare**® **PS 10** Sodium Laureth Sulfate (and) Coco Glucosides | | | 20,0 | |
| **Dehyton**® **PK 45** Cocamidopropyl Betaine | | 10,0 | 10,0 | |
| **Lamesoft® PO 65** Coco-Glucosid (and) Glyceryl Oleate | 5,0 | | | |
| **Lamesoft® LMG** Glyceryl Laurate (and) Potassium Cocoyl Hydrolyzed Collagen | | 5,0 | 5,0 | |
| **Gluadin**® **WQ** Laurdimonium Hydroxapropyll Hydrolyzed Wheat Protein | 3,0 | | | |
| **Gluadin**® **WK** Sodium Cocoyl Hydrolyzed Wheat Protein | | | | |
| **Euperlan® PK 3000 AM** Glycol Distearate (and) Laureth-4 (and) Cocamidopropyl Betaine | 5,0 | 3,0 | 4,0 | - |
| **Panthenol** | 0,5 | - | - | 0,5 |
| **Extrakt nach Beispiel 1 bis 3** | 1,0 | 1,0 | 1,0 | 1,0 |
| **Arlypon**® **F** Laureth-2 | 2,6 | 1,6 | - | 1,0 |
| **Sodium Chloride** | - | - | - | - |

**Tabelle 10**

| **Kosmetische Zubereitungen Schaumbad (Wasser, Konservierungsmittel ad 100 Gew.-%) - Fortsetzung 2** | | | | | |
|---|---|---|---|---|---|
| **Zusammensetzung** Bezeichnung nach INCI | **9** | **10** | **11** | **12** | **12** |
| **Texapon® NSO** Sodium Laureth Sulfate | - | 30,0 | 30,0 | - | 25,0 |
| **Plantacare® 818** Coco Glurosides | - | 10,0 | - | - | 20,0 |
| **Plantacare**® **PS 10** Sodium Laureth Sulfate (and) Coco Glucosides | 22,0 | - | 5,0 | 22,0 | - |
| **Dehyton**® **PK 45** Cocamidopropyl Betaine | 15,0 | 10,0 | 15,0 | 15,0 | 20,0 |
| **Monomuls® 90-018** Glyceryl Oleate | 0,5 | | | | |
| **Lamesoft® PO 65** Coco-Glucosid (and) Glyceryl Oleate | | 3,0 | | 3,0 | |
| **Cetiol® HE** PEG-7 Glyceryl Cocoate | | | 2,0 | | 2,0 |
| **Nutrilan® I-50** Hydrolyzed Collagen | 5,0 | | | | |
| **Gluadin® W 40** Hydrolyzed Wheat Gluten | | 5,0 | | 5,0 | |
| **Gluadin® WK** Sodium Cocoyl Hydrolyzed Wheat Protein | | | | 7,0 | |
| **Euperlan® PK 3000 AM** Glycol Distearate (and) Laureth-4 (and) Cocamidopropyl Betaine | 5,0 | - | - | 5,0 | - |
| **Arlypon® F** Laureth-2 | | | 1,0 | | |
| **Sodium Chloride** | 1,0 | | 1,0 | | 2,0 |
| **Extrakt nach Beispiel 1 bis 3** | 1,0 | 1,0 | 1,0 | 1,0 | 1,0 |

## Patentansprüche

1. Kosmetische Verwendung von Extrakten des Pilzes Grifola frondosa in Pflegemitteln zur kosmetischen Behandlung von Alterserscheinungen der Haut.

2. Kosmetische Verwendung nach Anspruch 1, wobei durch UV-Strahlung induzierte Alterserscheinungen der Haut behandelt werden.

3. Kosmetisches Sonnenschutzmittel enthaltend Extrakte des Pilzes Grifola frondosa und mindestens eine Verbindung ausgewählt aus der Gruppe bestehend aus
• 3-Benzylidencampher, 3-Benzylidennorcampher, 3-(4-Methylbenzyliden) campher ,
• 4-(Dimethylamino)benzoesäure-2-ethylhexylester, 4-(Dimethylamino) benzoesäure-2-octylester, 4-(Dimethylamino)benzoesäureamylester,
• 4-Methoxyzimtsäure-2-ethylhexylester, 4-Methoxyzimtsäurepropylester, 4-Methoxy-zimtsäureisoamylester 2-Cyano-3,3-phenylzimtsäure-2-ethyl-hexylester (Octocrylene),
• Salicylsäure-2-ethylhexylester, Salicylsäure-4-isopropylbenzylester, Salicylsäurehomomenthylester,
• 2-Hydroxy-4-methoxybenzophenon, 2-Hydroxy-4-methoxy-4'-methylbenzophenon, 2,2'-Dihydroxy-4-methoxybenzophenon,
• 4-Methoxybenzmalonsäuredi-2-ethylhexylester,
• 2,4,6-Trianilino-(p-carbo-2'-ethyl-1'-hexyloxy)-1,3,5-triazin, Octyl Triazon, Dioctyl Butamido Triazone,
• 1-(4-tert.Butylphenyl)-3-(4'methoxyphenyl)propan-1,3-dion,
• 2-Phenylbenzimidazol-5-sulfonsäure, deren Alkali-, Erdalkali-, Ammonium-, Alkylammonium-, Alkanolammonium- und Glucammoniumsalze,
• 2-Hydroxy-4-methoxybenzophenon-5-sulfonsäure, ihre Salze;
• 4-(2-Oxo-3-bornylidenmethyl)benzolsulfonsäure, 2-Methyl-5-(2-oxo-3-bornyliden) sulfonsäure und deren Salze,
• 1-(4'-tert.Butylphenyl)-3-(4'-methoxyphenyl)propan-1,3-dion, 4-tert.-Butyl-4'-methoxydibenzoylmethan,
• 1-Phenyl-3-(4'-isopropylphenyl)-propan-1,3-dion,
• Zinkoxid, Titandioxid, Oxide des Eisens, Zirkoniums, Siliciums, Mangans, Aluminiums und Cers, Silicate, Bariumsulfat und Zinstearat.

4. Ein Extrakt des Pilzes Grifola frondosa zur therapeutischen oder prophylaktischen Anwendung als Sonnenschutzmittel.

## Claims

1. Cosmetic use of extracts of the fungus Grifola frondosa in care preparations for the cosmetic treatment of signs of skin ageing.

2. Cosmetic use as claimed in claim 1, **characterized in that** UV-induced signs of skin ageing are treated.

3. Cosmetic sun protection preparations containing extracts of the fungus Grifola frondosa and at least one compound selected from the group consisting of
• 3-benzylidene camphor, 3-benzylidene norcamphor, 3-(4-methylbenzylidene)-camphor,
• 4-(dimethylamino)-benzoic acid-2-ethylhexyl ester, 4-(dimethylamino)-benzoic acid-2-octyl ester, 4-(dimethylamino)-benzoic acid amyl ester,
• 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene),
• salicylic acid-2-ethylhexyl ester, salicylic acid-4-isopropylbenzyl ester, salicylic acid homomenthyl ester,
• 2-hydroxy-4-methoxybenzophenone, 2-hydroxy-4-methoxy-4'-methylbenzophenone, 2,2'-dihydroxy-4-methoxybenzophenone,
• 4-methoxybenzalmalonic acid di-2-ethylhexyl ester,
• 2,4,6-trianilino-(p-carbo-2'-ethyl-1'-hexyloxy)-1,3,5-triazine, Octyl Triazone, Dioctyl Butamido Triazone,
• 1-(4-tert.butylphenyl)-3-(4'-methoxyphenyl)-propane-1,3-dione,
• 2-phenylbenzimidazole-5-sulfonic acid and alkali metal, alkaline earth metal, ammonium, alkylammonium, alkanolammonium and glucammonium salts thereof,
• 2-hydroxy-4-methoxybenzophenone-5-sulfonic acid and salts thereof,
• 4-(2-oxo-3-bornylidenemethyl)-benzene sulfonic acid, 2-methyl-5-(2-oxo-3-bornylidene)-sulfonic acid and salts thereof,
• 1-(4'-tertbutylphenyl)-3-(4'-methoxyphenyl)-propane-1,3-dione, 4-tert.butyl-4'-methoxydibenzoyl methane,
• 1-phenyl-3-(4'-isopropylphenyl)-propane-1,3-dione,
• zinc oxide, titanium dioxide, oxides of iron, zirconium, silicon, manganese, aluminium and cerium, silicates, barium sulfate and zinc stearate.

4. An extract of the fungus Grifola frondosa for therapeutic or prophylactic use as a sun protection preparation.

## Revendications

1. Utilisation cosmétique d'extraits du champignon Grifola frondosa dans des agents de soin pour le traitement cosmétique de manifestations du vieillissement cutané.

2. Utilisation cosmétique selon la revendication 1, par laquelle on traite des manifestations du vieillissement cutané induites par le rayonnement UV.

3. Agents cosmétiques de protection anti solaire renfermant des extraits du champignon Grifola frondosa et au moins un composé choisi dans le groupe constitué par :
- le 3-benzylidène camphre, le 3-benzylidène-norcamphre, le 3-(4-méthylbenzylidène) camphre,
- le 2-éthylhexylester d'acide 4-(diméthylamino) benzoïque, le 2-octylester d'acide 4-(diméthylamino) benzoïque, l'amylester d'acide 4-(diméthylamino) benzoïque,
- le 2-éthylhexylester d'acide 4-méthoxy cinnamique, le propylester d'acide 4-méthoxy cinnamique, l'isoamylester d'acide 4-méthoxy cinnamique, le 2-éthylhexylester d'acide 2-cyano-3,3 phénylcinnamique (octocrylène),
- le 2-éthylhexylester d'acide salicylique, le 4-isopropylbenzylester d'acide salicylique, l'homomenthylester d'acide salicylique,
- la 2-hydroxy-4-méthoxybenzophénone, la 2-hydroxy-4-méthoxy-4'-méthylbenzophénone, la 2,2'-dihydroxy-4-méthoxybenzophénone,
- le di-2-éthylhexylester d'acide 4-méthoxybenzomalonique,
- la 2,4,6-trianilino-(p-carbo-2'-éthyl-1'-hexyloxy)-1,3,5-triazine, l'octyl triazone, la dioctyl butamido triazone,
- la 1-(4-tert.butylphényl)-3-(4'méthoxyphényl)propane-1,3-dione,
- l'acide 2-phénylbenzimidazol-5-sulfonique, ses sels alcalins, alcalino terreux, d'ammonium, d'alkylammonium, d'alcanolammonium et de glucammonium,
- l'acide 2-hydroxy-4-méthoxybenzophénone-5 sulfonique et ses sels,
- l'acide 4-(2-oxo-3-bornylidéneméthyl)benzènesulfonique, l'acide 2-méthyl-5-(2-oxo-3-bornylidène) sulfonique et leurs sels,
- la 1-(4'-tert.butylphényl)-3-(4'-méthoxyphényl)propane-1,3-dione, le 4-tert.butyl-4'-méthoxydibenzoylméthane,
- la 1-phényl-3-(4'-isopropylphényl)-propane-1,3-dione,
- l'oxyde de zinc, l'oxyde de titane, les oxydes de fer de zirconium, de silicium, de manganèse, d'aluminium et de cérium, les silicates, le sulfate de baryum et le stéarate d'étain.

4. Un extrait du champignon Grifola frondosa pour une utilisation thérapeutique ou prophylactique en tant qu'agent de protection anti solaire.
